# EUROPEAN PATENT APPLICATION

(11) **EP 4 029 569 A1**
(43) Date of publication of application: **20.07.2022**
(21) Application number: 21211817.8
(22) Date of filing: 01.12.2021
(51) Int. Cl.: A61P 31/14, C07K 16/10, C12N 9/24, C12N 9/28, C12N 15/82

(54) **ENGINEERING, PRODUCTION AND CHARACTERIZATION OF PLANT PRODUCED NUCLEOCAPSID AND SPIKE STRUCTURAL PROTEINS OF SARS-COV-2 AS VACCINE CANDIDATES AGAINST COVID-19**

(30) Priority: 02.12.2020 US 202063120223 P
(71) Applicant: Mammedov, Tarlan, 07070 Konyaalti / Antalya (TR); Gulnara, Hasanova, 07070 Antalya (TR)
(72) Inventor: Mammedov, Tarlan, 07070 Konyaalti / Antalya (TR); Gulnara, Hasanova, 07070 Antalya (TR)
(74) Representative: Kancelaria Eupatent.pl Sp. z.o.o

(57) **Abstract**

This document relates to materials and methods for engineering and production of highly soluble, functional active recombinant Nucleocapsid (N) and Spike (S) based vaccine candidates against highly pandemic SARC-CoV-2 infection, in *Nicotiana benthamiana* plant using a transient expression system.

## Description

### TECHNICAL FIELD

This document relates to materials and methods for engineering and production of highly soluble, functional active recombinant Spike (S) and N (Nucleocapsid) of SARC-CoV-2 based antigens in *Nicotiana benthamiana* plants using a transient expression system, as vaccine candidates against highly pandemic SARC-CoV-2. Two forms, glycosylated and deglycosylated variants of Receptor Binding Domain (RBD) were produced in *Nicotiana benthamiana* plants. In addition, an antigen cocktail, comprising N protein and RBD of Spike protein of SARS-CoV-2, was invented and produced by co-expression of N protein and RBD of S protein of SARS-CoV-2 in N. benthamiana plant, for the first time, as a vaccine candidate, which may have broader-spectrum protection.

### SUMMARY OF THE INVENTION

This document relates to materials and methods for engineering and production of highly soluble, functional active recombinant Nucleocapsid (N) and Spike (S) based vaccine candidates against highly pandemic SARC-CoV-2 infection, in *Nicotiana benthamiana* plant using a transient expression system. A number of studies have now shown that CoV spike (S) glycoprotein and nucleocapsid (N) proteins promising targets for vaccine, antibodies and therapeutic drug development against deadly, pandemic SARS-CoV-2. The Nucleocapsid (N) and S protein variants (Receptor binding domain, RBD), of SARS-CoV-2, was engineered and produced in *Nicotiana benthamiana* plant. Under this invention the following vaccine antigens against SARS-CoV-2 infection were developed: i) RBD based vaccine antigens; two forms, glycosylated and deglycosylated variants of RBD were produced in *Nicotiana benthamiana* plants (see Mamedov et al., 2021a). Endo H *in vivo* deglycosylated RBD exhibited a stronger binding to ACE2 compared with its glycosylated counterpart. In addition, sera immunized with deglycosylated RBD displayed high virus neutralizing activity compared to that of glycosylated RBD variant. The expression levels of gRBDor dRBD proteins were more than 40 mg/kg plant leaf, which would be sufficient for commercialization of these vaccine candidates. The design and correct selection of the amino acid region of RBD was critical for high-yield production of a functionally active and soluble RBD protein. Notably, other researchers producing RBD-based vaccines had very low expression level and low activity due to incorrect selection of amino acid regions of the RBD of SARS-CoV-2. This was discussed extensively in our published article on the development of glycosylated and deglycosylated RBD-based SARS-CoV-2 vaccine candidates. (Mamedov et al., 2021a); ii) Within the scope of this invention, the antigen cocktail containing N protein and RBD (N+RBD) was invented and produced in the *N. benthamiana* plant for the first time. The idea of antigen cocktail was created and antigen cocktail was produced by co-expression of the RBD of S and N proteins of SARS-CoV-2 in *N. benthamiana* plant as vaccine candidate against SARS-CoV-2 infection, for the first time. N protein has been shown to be highly immunogenic and, most importantly, more conserved compared to the S protein among SARS-CoV-2 variants and other coronaviruses. It shares 91% similarity with SARS-CoV, in addition to greater stability with fewer observed mutations (Mamedov et al., 2021a, 2021b). In this regard, our hypothesis was that N protein with a conserved sequence may provide additional and broader protection against possible variants of SARS-CoV-2. Although plant-produced N protein did not induce neutralizing responses in mice, our hypothesis was that an antigen cocktail may generate a durable immune response and additional protective effects compared to RBD alone. In fact, the cocktail antigen elicited high-titer functional antibodies compared to RBD or N proteins. Thus, N, RBD, N+RBD (cocktail) antigens are promising vaccine candidates against COVID-19. In addition, the multi-antigen vaccine approach, developed under this invention can be applied to existing COVID-19 vaccines, in particular mRNA, DNA, viral vectors, and other types of vaccines, to be effective toward new emerging variants.

### BACKGROUND

The novel coronavirus, currently designated as SARS-CoV-2, is a novel and highly pathogenic coronavirus, and spread to more than 220 countries and territories for a short time, and as of November 18 2021, more than 254,256,432 cases were recorded, and more than 5,112,461 people were killed. Vaccination is the only way to prevent infectious diseases, control a pandemic, and reduce morbidity and mortality. Although a number of vaccines (mRNA, adenovirus vector vaccines, and inactivated vaccines) have been approved (22 approved vaccines, see: https://covid19.trackvaccines.org/vaccines/approved/#vaccine-list accessed on 16 September 2021) and are currently used for mass vaccinations, the effectiveness of these vaccines is diminishing due to the emergence of new and recent strains, especially the Delta strain (B.1.617), which has been found in many countries and continues to spread rapidly around the world. Therefore, the world still urgently needs more effective COVID-19 vaccines with a stable immune response and long-term immunity against possible emerging mutated variants of SARS-CoV-2 viruses. Since the S protein of SARS-CoV, MERS-CoV, and SARS-CoV-2 contains neutralizing epitopes, it has been shown to be a leading candidate for vaccine developments. In this regard, a number of studies demonstrated that the S protein induced potent humoral and cellular immune responses in tested animal models. Most COVID-19 vaccines, which are currently approved or under development (mRNA, DNA, viral vector-based, subunits, and protein-based vaccine) were designed and developed on the basis of the S protein (see Mamedov et al., 2021). When reported in January 2020, in spike protein, SARS-CoV-2 had about ~80% sequence identity with that of SARS-CoV (see Mamedov et al., 2021a). Now the sequence identity is less than 75% as the spike protein of SARS-CoV-2 has acquired more than 725 mutations.

Under this invention two forms of RBD of Spike protein of SARC-CoV-2 were produced in plants and most pre-clinical tests were completed. Under this invention, for the first time, deglycosylated form of RBD was produced in N. benthamiana plant (Mamedov et al., 2021a). Prior to submitting our current patent application, one group published the RBD of the S protein of SARC-CoV-2 in plants (Rattanapisit et al., 2020), however, the selection of amino acid regions of RBDs in this study was not properly designed. As such, amino acids of F318-C617 was designed where cysteine at position 617 remained unpaired, which forms a disulfide bridge with C649 in the full-length S protein (see Mamedov et al., 2021a). Notably, S protein of SARS-CoV-2 is cysteine-rich and nine cysteine residues are found in the RBD, eight of which are involved in forming four pairs of disulfide bridges Therefore, very low RBD expression (8 mikrogram/g plants) was obtained in this study. Furthermore, no functional activity such as virus neutralizing activity was reported in this study. An unsatisfactory expression level (2-4 mikrogram/g fresh weight) was also observed for His-tagged RBD variant (aa R319-F541), reported by Diego-Martin et al. (2021) and Shin et al. (2021), which was too low to be economical for commercialization. These studies were reported after our provisional patent application was filed. Thus, this to produce functional active RBD based vaccine against COVID-19 would not be obvious, Based on a number of studies, it has been found that the selection of amino acid regions of RBD is critical for the correct formation of the disulfide bridge, therefore, for proper folding of the resulting recombinant SARS-CoV-2 RBD.

Under this invention, we developed N protein and an antigen cocktail comprising RBD and N proteins of SARS-CoV-2 as vaccine candidates against SARS-CoV-2 infection (see Mamedov et al., 2021b). The antigen cocktail was produced by co-expression of the RBD of S protein and N proteins of SARS-CoV-2 in N. benthamiana plant. N protein has been shown to be highly immunogenic and, most importantly, more conserved among SARS-CoV-2 variants and other coronaviruses (Maharjan et al, 2021). In fact, fewer mutations have been observed in the N protein over time (Grifoni et al., 2020; Mamedov et al., 2020). It has also been recently shown that the crystal structure of the SARS-CoV-2 nucleocapsid protein (Kang et al., 2020) is very similar to coronavirus N proteins, which were previously described. Cong et al. (2020) using a mouse hepatitis virus model showed that the nucleocapsid protein contributes to forming helical ribonucleoproteins during the packaging of the RNA genome, thereby regulating viral RNA synthesis during replication and transcription (Cong et al.,2020). A number of studies have shown the critical roles of N protein at multiple stages of the viral lifecycle (Maharjan et al., 2021) It was also demonstrated that N proteins of many coronaviruses are highly immunogenic and are produced abundantly during infection, and high levels of IgG antibodies against N protein have been detected in sera from patients who recovered from SARS (Leung et al., 2004). Under this invention, we show that plant-produced N and N+RBD antigens were able to induce significantly high titers of antibodies with Alhydrogel adjuvant with potent. Although plant-produced N protein did not induce neutralizing responses in mice, our hypothesis is that an antigen cocktail may generate a durable immune response and additional protective effects compared to RBD alone.

### OBJECT OF THE INVENTION

SARS-CoV-2 is a novel and highly pathogenic coronavirus, which has caused an outbreak in Wuhan city, China in 2019, and then soon spread nationwide and spilled over to other countries and the world. Head of the United Nations has described this as humanity's worst crisis since World War II. Although various types of vaccines (mRNA, adenovirus vector vaccines, and inactivated vaccines) are now available and some other ones (mRNA, DNA, and viral vector-based, protein based subunits, inactivated, attenuated) are underway, the world still urgently needs more safe and effective, alternative SARS-CoV-2 vaccines, antiviral and therapeutic drugs, cost- effective diagnostic reagents and kits to control the COVID-19 pandemic and relieve the human suffering associated with the pandemic that kills thousands of people every day. As SARS-CoV-2 mutations appear worldwide, the effectiveness of existing vaccines against variants is declining. The objective of this invention was to produce cost-effective, safe, highly immunogenic vaccine antigens against SARS-CoV-2, more importantly, vaccines being effective to existing variants of SARS-CoV-2. Notably, among a number of expression systems, transient expression in plants is a promising platform for the production of recombinant proteins. This system allows the cost-effective production of a variety of complex recombinant proteins, including vaccine candidates, therapeutic proteins, enzymes, and antibodies, in a short period of time, with high yield and fully functional activity. In this study, we engineered and produced Spike protein and N protein based vaccine antigens in *N. benthamiana* plant as follow:
1. Receptor binding domain of Spike protein of SARS-CoV-2 (RBD) (AA 319- 591, as Flag or His6 tagged variants, GenBank: QHO60594.1). Glycosylated and Endo H in vivo deglycosylated forms of RBD proteins of SARS-CoV-2 were produced in *N. benthamiana* plant. A deglycosylated RBD variant was produced by co-expression of the SARS-CoV-2 RBD gene with bacterial Endo H-the in vivo deglycosylation strategy that we recently developed (Patent No .: US 11,041,163 B2). Under this invention, for the first time, deglycosylated form of RBD was reported (see Mamedov et al., 2021a).
2. N gene (full length, AA 1-419, GenBank accession YP_009724397, as Flag tagged) of SARS-CoV-2.
3. Nucleocapsid (N) protein co-expressed with RBD of SARS-CoV-2 in N. benthamiana plant to produce an antigen cocktail. As part of this invention, a multi-antigen cocktail vaccine against SARS-CoV-2 has been developed and produced for the first time as a promising new type of vaccine candidate against COVID-19. As mentioned above, as SARS-CoV-2 mutations appear worldwide, the effectiveness of existing vaccines against variants is declining. Flexible approaches are required for the production of vaccines against COVID-19 that are effective against emerging variants. The N-protein + RBD multi-antigen vaccine approach that we proposed, designed, and produced for the first time, which was widely discussed in a recently published review article (Maharjan et al., 2021), could be a promising approach for the production of effective vaccines against emerging SARS-CoV-2 variants, given that the N-protein is highly immunogenic, more conserved, and less vulnerable to possible emerging mutations. The multi-antigen vaccine approach can be applied to existing COVID-19 vaccines, in particular mRNA, DNA, viral vectors, and other types of vaccines, to be effective toward new emerging variants.

### Industrial Application of the Invention

Under this invention several COVID-19 antigens (N protein, RBD protein, N+RBD) were produced in plants. The purification yields were more that 20 mg of pure protein/kg of plant biomass for each target antigen, which would be sufficient for commercialization of these vaccine candidates. Thus, plant produced antigens, designed, developed and produced in plants under this invention can be used as potential vaccines against COVID-19. As SARS-CoV-2 mutations appear worldwide, the effectiveness of existing vaccines against variants is declining. All our data support that plant-produced glycosylated and deglycosylated RBD antigens are promising vaccine candidates for the prevention of COVID-19. The N-protein + RBD multi-antigen vaccine approach that we proposed, designed, and produced for the first time (Mamedov et al., 2021) could be a promising approach for the production of effective vaccines against emerging SARS-CoV-2 variants, given that the N-protein is highly immunogenic, more conserved, and less vulnerable to possible emerging mutations. The multi-antigen vaccine approach can be applied to existing COVID-19 vaccines, in particular mRNA, DNA, viral vectors, and other types of vaccines, to be effective toward new emerging variants. Plant-produced N, RBD, N+RBD and S1 antigens can also be used as a diagnostic reagent in serological tests for detection of SARS-COV-2 antibodies in COVID-19 patients.

### Example 1 - Materials and Methods

### Cloning and expression of Nucleocapsid (N) and Spike (S) protein variants in N. benthamiana. T

The nucleocapsid gene (1-419 aa GenBank accession YP_009724397) and spike gene of SARS-CoV-2 variant RBD (receptor-binding domain containing fragment, RBD, 319-591 aa, GenBank accession MN985325, Flag- or His6-tagged) and nd S1 domain (AA 14-815, GenBank accession MN985325, as a His6 tagged) were optimized for expression in N. benthamiana plants and de novo synthesized at Biomatik Corp. (Kitchener, ON, Canada). To transiently express N, RBD, or N+RBD variants in N. benthamiana plants, the Nicotiana tabacum PR-1a signal peptide (MGFVLFSQLPSFLLVSTLLLFLVISHSCRA) was added to the N-terminus of N and RBD proteins. In addition, the KDEL sequence (the ER retention signal) and the FLAG epitope (the affinity purification tag for N and RBD proteins) were added to the C-terminus. The resulting sequences were inserted into the pEAQ binary expression vectors to obtain pEAQ-N and pEAQ-RBD. These plasmids were then transferred into the Agrobacterium AGL1 strain. To express N, RBD, or N+RBDN+RBD variants in N. benthamiana plant, AGL1 harboring pEAQ-N and pEAQ-RBD plasmids was infiltrated into N. benthamiana plant leaves. To co-express N and RBD proteins, pEAQ-N and pEAQ-RBD plasmids were infiltrated into plant leaves. Plants were harvested at 4 dpi (day after post infiltration). To co-express N and Endo H, AGL1 strain harboring pEAQ-N was co-infiltrated with pGreen-Endo H construct.

### Expression screening of RBD, N and N+RBD variants produced in N. benthamiana plant by Western blot analysis.

SDS-PAGE analysis of plant produced RBD, N and N+RBD variants were performed on 10% acrylamide gels stained with Coomassie (Gel Code Blue, Pierce Rockford, IL). Western blot analysis was performed after electrophoresis and transfer of the proteins to Polyvinylidene Fluoride membranes. After transfer, Western blot membranes were blocked with I-Block (Applied Biosystems, Carlsbad, CA) and recombinant Flag-tagged N, RBD and N+RBD proteins were detected with an anti-FLAG antibody and anti-RBD of S protein of SARC-CoV-2 monoclonal antibody (cat. no. MBS2563882, MyBiosource) or Human Novel Coronavirus Nucleoprotein (N) (1-419aa) monoclonal Antibody (MyBioSource, cat. no. MBS7135930).

### In vivo production of deglycosylated version of RBD protein by co-expression with Endo H

To co-express a FLAG tagged S1 protein with Endo H, pEAQ-S1 and pGeen-Endo H (Mamedov et al., 2017; WO2017081520A1, Patent No .: US 11,041,163 B2) constructs were used for plant co-infiltration.

### Purification of plant produced N, RBD and N+RBD proteins using anti-DYKDDDDK affinity gel

Purification of plant produced N and RBD (glycosylated and deglycosylated) and N+gRBD variants were performed by anti-FLAG affinity chromatography using anti-DYKDDDDK affinity gel. For purification, 20 g of frozen leaves, infiltrated with the pEAQ-N-Flag-KDEL or pEAQ-S1- Flag-KDEL constructs were ground in 20 mL PBS buffer (1XPBS, 150 mM NaCI) using a mortar and a pestle. Plant debris was removed by filtration through Miracloth followed by centrifugation at 20,000 g for 25 minutes and then filtered through a 0.45 µm syringe filter (Millipore). An anti- FLAG affinity column was prepared according to the manufacturer's instructions. Sixty milliliters of a clear supernatant were loaded into 0.5 ml resin column equilibrated with PBS buffer. The column was washed with 10 volumes of PBS buffer. Bound proteins were eluted using 200 mM Glycine, 150 mM NaCl, pH 2.2 into tubes containing 2.0 M Tris solution to neutralize. Total protein content was estimated using the BioDrop and then analyzed by SDS-PAGE and western blot.

### Gel Filtration

Gel filtration of plant-produced N and N+RBD proteins was performed with ÄKTA start using a 60 cm _ 16 mm column (cat. no. 19-5003-01, GE Healthcare, Chicago, IL, USA), packed with Sephacryl^{®} S-200 HR (cat. no. 17-0584-10, GE Healthcare), as described previously [4]. The column was first equilibrated with 50 mM phosphate buffer, pH 7.4, 150 mM sodium chloride, and then 0.25 mg of plant-produced N and N+gRBD proteins, purified using FLAG affinity chromatography, were loaded onto a column. All eluted fractions from the column were combined and concentrated, buffer exchanged against PBS buffer, concentrated using a Millipore 10K MWCO Amicon Ultra 4 concentrator (cat. no.: UFC8010, Millipore), and analyzed by SDS-PAGE and Western blot analyses.

### Immunogenicity studies of N, RBD, N+RBD, and S1 protein in mice

Mice received two doses of N, S1 and N+S1 (Cocktail) proteins adsorbed to 0.3% Alhydrogel at three-week intervals (0, 21 days). Groups of seven-week-old mice (6 animals/group) were immunized IM with 5 µg of N, S1 or N+S1 variants at 0 and 21 days. Serum samples were collected on days -1 (pre-bleed) and 42 (post 2nd vaccination) and assessed for anti-N, S or anti-N+S1 antibody responses by an IgG ELISA. Mice studies were carried out at Akdeniz University Experimental Animal Care Unit under permission of the Local Ethics Committee for Animal Experiments at Akdeniz University with the supervision of a veterinarian.

### Development of N, RBD, N+RBD plant produced protein-based ELISA kits

The ELISA kit, has been developed in this study based on plant produced N, S1and S2 antigens to qualitatively and quantitatively detect the SARS-CoV-2 spike protein IgG in serum or plasma from patients infected with COVID-19. Elabscience Euroimmun ELISA Kits (SARS-CoV-2 Spike Protein IgG ELISA Kit, cat. no. E-EL-E602) was used as control for comparison. Blood samples were diluted in 100 fold. HRP Conjugated anti-human IgG (MyBioSource Inc., cat. no. MBS440121) were used to develop the antigen-antibody-HRP conjugated secondary antibody complex. 200 ng plant produced N, S1 or S2 antigens were coated into each wells. The plate was read at 450 nm on a multiwell plate reader.

### SDS-PAGE and Western blot analysis of purified N, RBD, N+RBD variants

SDS-PAGE analysis of plant produced S variants was performed on 10% acrylamide gels stained with Coomassie (Gel Code Blue, Pierce Rockford, IL). Western blot analysis was performed after electrophoresis and transfer of the proteins to Polyvinylidene Fluoride membranes. After transfer, Western blot membranes were blocked with I-Block (Applied Biosystems, Carlsbad, CA) and recombinant proteins detected with an anti-FLAG (N, S1 and N+S1) or anti-His Antibody (S2), or anti-SARS-COV2 COVID 19 Spike Protein Coronavirus Monoclonal Antibody (MyBioSource, Inc, cat. no. MBS2563837). The image was taken using high sensitive GeneGnome XRQ Chemiluminescence imaging system (Syngene, A Division of Synoptics Ltd).

### Example 2- Engineering, Cloning, Expression, Purification, and Characterization of RBD Proteins from N. benthamiana

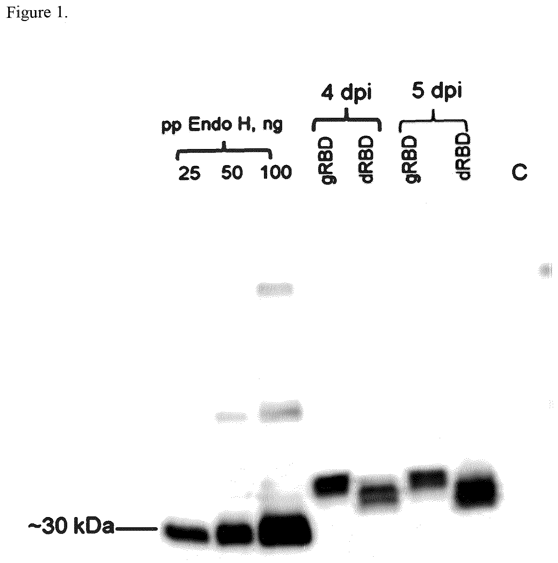

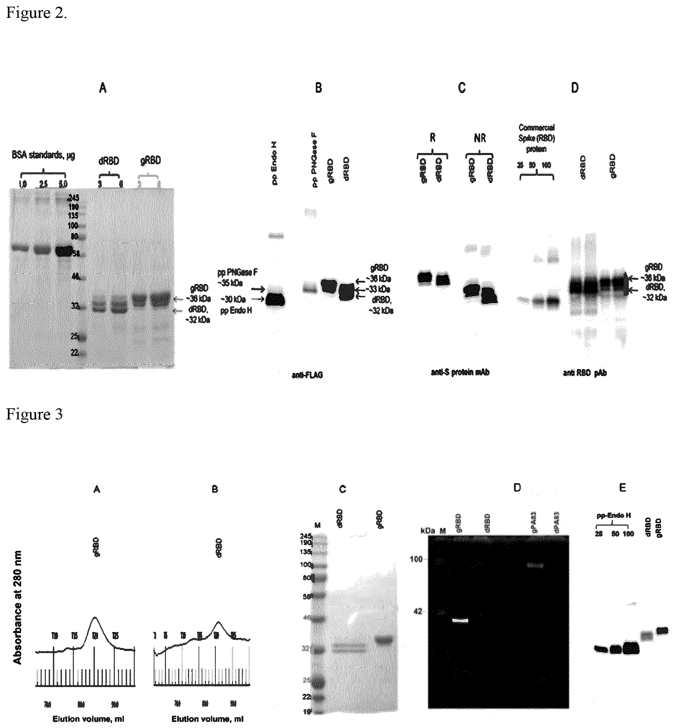

Both glycosylated and non-glycosylated variant of SARS-CoV-2 RBD was produced in the plant *N. benthamiana* (Fig. 1, The deglycosylated form of RBD was produced by co-expression of RBD gene of SARS-CoV-2 with bacterial deglycosylase Endo H (Patent No .: US 11,041,163 B2). The expression levels determined by Western blot analysis and ELISA were 42 mg/kg plant leaf for deglycosylated and 45 mg/kg for glycosylated forms of RBD. Glycosylated and deglycosylated plant-produced RBD proteins were purified from crude extracts by single-step anti-FLAG antibody affinity chromatography. The purification yields were 22 and 20 mg pure protein/kg plant biomass for gRBD and dRBD, respectively. SDS-PAGE and Western blot analysis of the anti-FLAG column purified deglycosylated and glycosylated variants of RBD are shown in Figure 2. The purity of both proteins was higher than 90%. Plant-produced, purified glycosylated RBD molecules appeared as a double protein with a molecular mass (MM) of ~33 or 36 kDa (Fig. 2, gRBD). dRBD molecules also appeared as a double protein with a MM of ~32 or ~33 kDa (Fig. 2, dRBD). The ~33 kDa band observed in the purified gRBD sample might be due to the presence of a deglycosylated form, observed when RBD was co-expressed with bacterial Endo H. Plant-produced dRBD and gRBD proteins were very well recognized by anti-FLAG (Fig. 2B) and by S protein-specific monoclonal (Fig. 2C) or polyclonal antibodies (Fig. 2D), demonstrating specific reactivity of SARS-CoV-2 specific mAb or pAb to the plant-produced gRBD and dRBD proteins. Both gRBD and Endo H *in vivo* deglycosylated RBD (dRBD) eluted as a single peak from the Sephacryl S-200 column and were present as monomers and no dimerization or aggregation were observed (Fig. 3A, B). Based on the SDS-PAGE analysis (Figure 3C), the purity of gRBD or dRBD proteins after gel filtration was higher than 95%. Sephacryl S-200 column-purified gRBD and dRBD protein were subjected to glycan detection to demonstrate the *in vivo* deglycosylation of target proteins by Endo H. As shown in Fig. 3D, the glycan was only detected in plant-produced glycosylated RBD and glycosylated PA83. Fig.3E shows the Western blot analysis of the same samples (diluted 1:5) probed with an anti-FLAG antibody.

### Example 3-Binding of Plant-Produced RBD Variants to ACE2

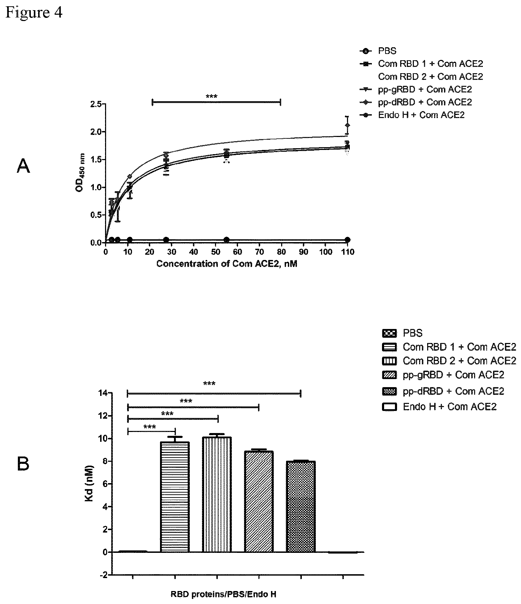

Binding of plant-produced gRBD and dRBD proteins, along with commercial RBDs, to ACE2 was assessed. As can be seen from Fig. 4, the plant-produced RBD variants demonstrated specific binding to ACE2, the SARS-CoV-2 receptor. Notably, Endo H *in vivo* deglycosylated RBD exhibited a slightly stronger binding (Kd = 7.88 ± 0.062 nM) to ACE2 compared with its glycosylated (Kd = 8.816 ± 0.054 nM) counterpart.

### Example 4- Immunogenicity Studies of gRBD and dRBD Variants in Mice

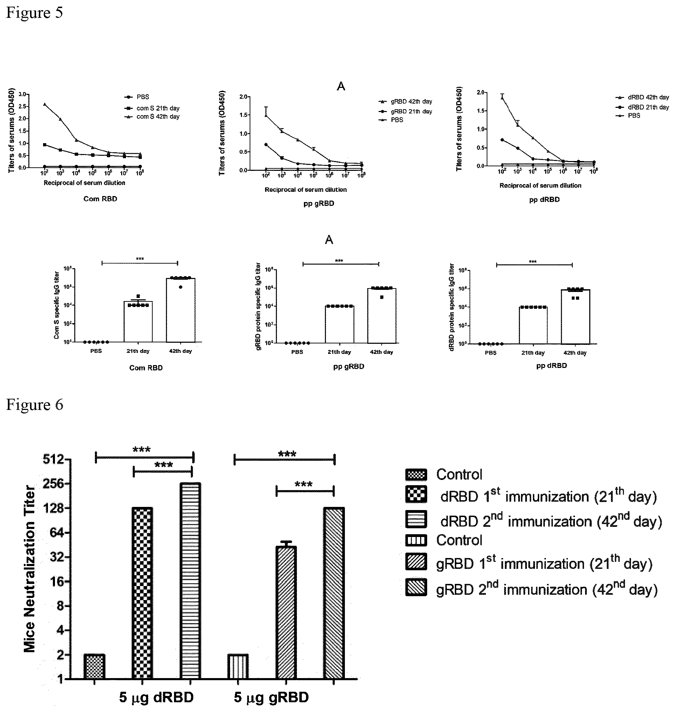

For immunogenicity studies mice received two doses of each variant, gRBD or dRBD proteins, adsorbed to 0.3% Alhydrogel at three-week intervals (0, 21 days). Serum samples were collected on days -1 (pre-bleed), 21 (post first vaccination), and 42 (post second vaccination) and assessed for anti-gRBD or anti-dRBD antibody responses with an IgG ELISA. IgG responses showed that plant-produced RBD protein variants were able to induce significantly high titers of antibodies with alum adjuvant at the 5 µg doses after 21 days and more after 47 days (Fig. 5).

### Example 5-Neutralization Activity Assessment of gRBD and dRBD by Micro Neutralization Test

The neutralization ability of sera from mice immunized with the plant-produced gRBD and dRBD proteins against live SARS-CoV-2 infection was examined in Vero-E6 cells. The neutralization titers for gRBD and dRBD are presented in Fig. 6. Sera collected from mice immunized with 5 µg plant-produced dRBD (adjuvanted with Alhydrogel), collected at day 21 post-first immunization, had a three-fold greater neutralization titer compared with plant-produced gRBD. Murine sera collected from mice immunized with 5 µg plant-produced gRBD or dRBD proteins (adjuvanted with Alhydrogel), collected at day 42 post-second immunization, displayed SARS-CoV-2 neutralization activity at 1:128 or 1:256 dilutions, respectively. Thus, both plant-produced SARS-CoV-2 RBD variants induced potent neutralizing responses in mice and sera immunized with the plant-produced SARS-CoV-2 dRBD variant had a greater neutralizing activity against live SARS-CoV-2 compared with its glycosylated counterpart, gRBD.

### Example 6-Engineering, Cloning and Expression, Purification, and Characterization of N Protein in N. benthamiana Plant

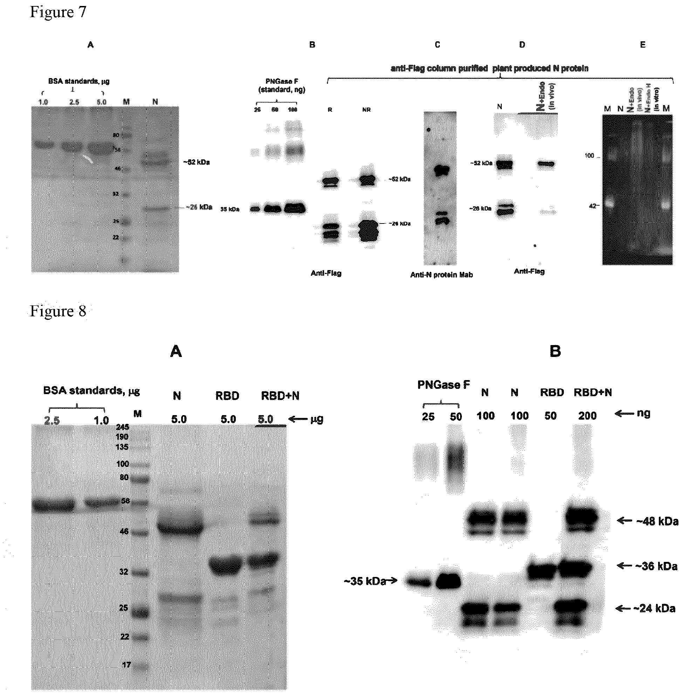

The expression level of N protein determined by Western blot analysis and ELISA was about 45 mg/kg of plant leaves. The plant-produced N protein was purified using single-step anti-FLAG affinity chromatography. The purification yield was about 22 mg pure protein/kg of plant biomass for Flag-tagged N protein. On SDS-PAGE and Western blotting, the plant-produced N protein molecule appeared as a doublet protein with an MM of ~48 and ~24 kDa (Fig. 7 A,B). Both molecular forms of plant-produced N proteins were recognized by anti-FLAG antibody (Fig. 7B) and by N protein-specific mAb (Fig. 7C), demonstrating specific reactivity of mAb to the two forms; it also confirmed that epitopes for mAb are still present in the ~24 kDa fragment. Fig. 7B demonstrates the SDS-PAGE followed by Western blotting of plant-produced N protein, performed under reducing and nonreducing conditions. The N protein of SARS-CoV-2 has five potential *N*-glycosylation sites. Therefore, we co-expressed the N protein gene with bacterial Endo H genes to confirm the *N*-glycosylation status of plant-produced N protein. When the N protein was co-expressed with Endo H, there was no protein band shift in the Western blotting (Fig. 7D). In addition, we performed glycan detection analysis of plant-produced N protein; no glycan was detected in plant-produced N protein (Fig. 7E), suggesting that plant-produced N protein is not *N*-glycosylated.

### Example 7- Co-Expression of N and gRBD, and Production and Purification of N+gRBD Antigens from N. benthamiana Plant. Gel Filtration Chromatography of N and N+RBD Proteins.

To produce antigen cocktails containing N+RBD proteins, agrobacterium harboring pEAQ-N and pEAQ-RBD constructs was infiltrated into plants, and plant leaves were harvested at 4 dpi (day of post infiltration). N protein and N+gRBD co-expressed (cocktail) protein were purified using single-step anti-FLAG affinity chromatography. SDS-PAGE (Fig. 8A) and Western blot (Fig. 8B) analysis of the cocktail protein purified from N. benthamiana plant demonstrated three major proteins with MM 48 kDa (N protein), 36 kDa (gRBD), and 24 kDa (N protein) in the cocktail antigens.

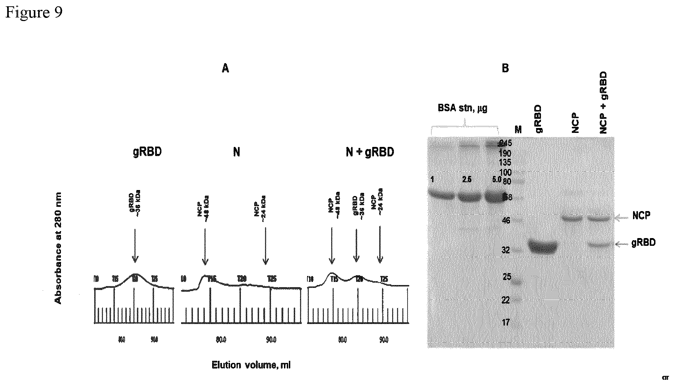

Anti-Flag affinity chromatography-purified N and N+RBD proteins were further analyzed by gel filtration through a Sephacryl S-200 column (Fig 9A). The N protein was eluted from the S-200 column as double peaks, and no dimerization or aggregation was observed. Similarly, N+gRBD proteins were eluted from the S-200 column as triple peaks, and no dimerization or aggregation was observed. Fig. 9B demonstrates the SDS-PAGE analysis of a full-length (~48 kDa) N protein, with antigen cocktails containing the ~48 kDa N protein and 36 kDa gRBD protein, eluted from the Sephacryl S-200 column and concentrated.

### Example 8-Stability Assessment of N Protein

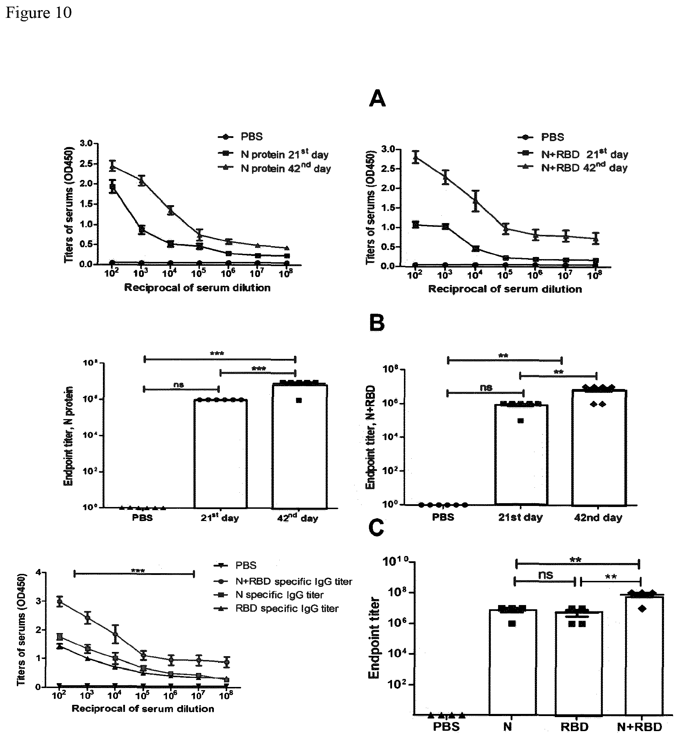

The stability analysis of plant-produced N protein (~48 kDa, full length) was examined after incubation at 37 °C for 24, 48, 96, 72, and 96 h. These analyses showed that the N protein was less than 50% degraded after incubation at 37 °C for 48 h (Fig.10A). There was almost no degradation of plant-produced N protein during a 6 month period when stored at -80 °C (Fig. 10B).

### Example 9- Immunogenicity Studies of N and N+RBD in Mice

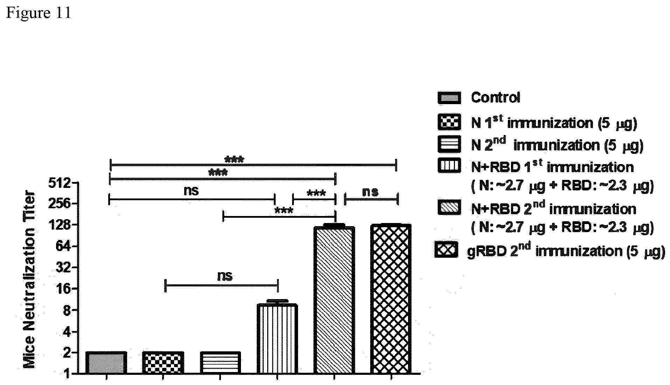

Antibody levels were determined by ELISA on 21st and 42nd day mouse sera immunized with two doses of N and N+RBD. As shown in Fig. 11A,B, the plant-produced N and N+RBD proteins were able to induce significantly high titers of antibody with a 5 µg dose. On the other hand, the endpoint titer of N+RBD obtained by co-expression of gRBD with N protein was 10⁷ in this study. The endpoint titer of gRBD was demonstrated to be 10⁶ (please see above). These immunogenicity analysis results demonstrate that N+RBD elicits high-titer antibodies compared to RBD or N proteins.

### Example 10- Neutralization Activity Assessment of N or N+RBD by Microneutralization Test

The neutralization activity of sera from mice immunized with the plant-produced, anti-FLAG affinity chromatography-purified N and N+gRBD proteins against live SARS-CoV-2 infection was further tested in Vero E6 cells. The results are presented in Fig. 12. Sera collected from mice immunized with 5 µg of plant-produced N protein (adjuvanted with Alhydrogel) collected on day 21 (after the first immunization) or 42 (after the second immunization) showed no neutralizing ability. Mice sera collected from mice immunized with 5 µg of plant-produced N+gRBD (adjuvanted with Alhydrogel) collected on day 21 (after the first immunization) or 42 (after the second immunization) displayed SARS-CoV-2 neutralization activity at 1:8 and 1:128, respectively. Murine sera, collected from mice immunized with 5.0 µg of plant-produced gRBD (alone), collected on day 42 (after the second immunization) displayed SARS-CoV-2 neutralization activity at 1:128, which is consistent with the results for RBD]. Similar neutralization activity (at 1:128) was observed with the sera of mice immunized with ~2.3 µg of gRBD but in combination with ~2.7 µg of N protein (~2.3 µg of gRBD + ~2.7 µg of N protein (total 5.0 µg) in immunized mice), demonstrating that the N protein possibly contributes to the enhancement of neutralizing activity.

### Example 11- Development of N, S1 and N+S1 (cocktail) plant produced protein-based ELISA kits

The ELISA kit has been developed in this study to qualitatively and quantitatively detect the SARS-CoV-2 S spike protein IgGi in serum or plasma from patients infected with COVID-19. The results of ELISA were shown in Fig. 13. As can be seen from Fig.13, plant produced S1 antigen is very well recognized by antibodies of plasma from COVID-19 patients. Plant produced N and S1+N antigens very also well recognized by antibodies of plasma from COVID-19 (data not shown). Importantly the data correlated with commercial available euroimmun kit. These data confirm that the ELISA kits, which were developed in this study, can be used to qualitatively and quantitatively detect the SARS-CoV-2 spike protein IgGi in serum or plasma from patients infected with COVID-19. These data provide visibility of the commercialization of these kits for serological testing.

### APPLICATION OF THE INVENTION

Based on characteristics of plant produced N, RBD, and N+RBD proteins antigens are good candidates for the development vaccines against COVID-19 vaccine. Another application of the invention is that plant produced N, RBD, and N+RBD antigens can be used as a ELISA kits for serological tests, to qualitatively and quantitatively detect the SARS-CoV-2 antibodies in serum or plasma from patients infected with COVID-19.

### DESCRIPTION OF DRAWINGS

**Figure 1** demonstrates Western blot analysis of the expression of RBD (receptor binding domain) and co-expression of RBD with bacterial Endo H in *N. benthamiana. N. benthamiana* plants, which were infiltrated with pEAQ-RBD or with pEAQ-RBD/pGreenII-Endo H (non-tagged) constructs, to produce glycosylated RBD (gRBD) or in vivo deglycosylated RBD (dRBD) of SARS-CoV-2, respectively. Leaf samples were collected at 4 dpi and 5 dpi (days post-infiltration) and were homogenized in three volumes of extraction buffer. RBD protein bands were probed using the anti-4×His tag mAb. Purified plant-produced FLAG-tagged Endo H protein (~30 kDa, 25, 50, and 100 ng) was used as a standard protein. C-crude extract prepared from a control, non-infiltrated plant. gRBD-glycosylated RBD; dRBD-deglycosylated RBD; pp-plant produced

**Figure 2** demonstrates **a** SDS-PAGE analysis of purified plant-produced gRBD and dRBD proteins. RBD protein variants were purified from *N. benthamiana* using anti-FLAG antibody resin. **(A)** gRBD-3 or 6 µg plant-produced glycosylated RBD; dRBD-3 or 6 µg plant-produced deglycosylated RBD; BSA standards-1.0, 2.5 and 5.0 µg; M-color prestained protein standard (NEB). **(B)** Western blot analysis of gRBD and dRBD using anti-Flag antibody: pp Endo H-plant-produced and purified Endo H protein with molecular mass of ~30 kDa (Mamedov et al., 2017). pp PNGase F-plant-produced and purified PNGase F protein with a molecular mass of ~35 kDa (Mamedov et al., 2017)]. **(C)** Western blot analysis of gRBD and dRBD using commercially available, purified anti-SARS-CoV-2 S protein S1 mAb (cat. no. 945102, BioLegend). **(D)** Western blot analysis of gRBD and dRBD using commercially available anti-RBD antibody (cat. no. MBS2563840, MyBiosource, San Diego, CA, USA); commercial spike protein-COVID 19 spike protein (RBD) active protein, sequence positions Arg319-Phe541 (MBS2563882, MyBiosource); pp-plant-produced; R-reducing; NR-nonreducing condition.

**Figure 3** demonstrates a Gel filtration chromatography and glycan detection of plant-produced gRBD or dRBD proteins. Profiles of plant-produced gRBD **(A)** and dRBD **(B)** proteins, eluted from Sephacryl^{®} S-200 HR column. The column was equilibrated with 50 mM phosphate buffer (with 150 mM NaCl, pH 7.4). The plant-produced dRBD and gRBD proteins (0.25 mg), purified using FLAG affinity chromatography, were loaded onto column. Gel filtration was performed with ÄKTA start using 60 cm × 16 mm column (cat. no. 19-5003-01, GEHealthcare, Chicago, IL, USA), packed with Sephacryl^{®} S-200 HR (cat. no. 17-0584-10, GE Healthcare). **(C)** SDS-PAGE analysis of plant-produced gRBD and dRBD proteins eluted from Sephacryl^{®} S-200 HR column. **(D)** Glycan detection in gRBD and dRBD proteins. Protein (250 ng) from each sample (eluted from Sephacryl^{®} S-200 HR column) was separated on a 10% SDS-PAGE gel followed by in-gel glycan detection using the Pro-Q Emerald 300 glycoprotein staining kit. Stained proteins were visualized by UV illumination. M-CandyCane glycoprotein molecular weight standards (Molecular Probes), 250 ng of each protein per lane; gRBD-glycosylated RBD; dRBD-deglycosylated RBD; gPA83-glycosylated PA83; dPA83-deglycosylated PA83. **(E)** Western blot analysis of the same sample using the anti-FLAG antibody. Purified Endo H protein (25, 50, and 100 ng) and gRBD or dRBD proteins (50 ng) were loaded into wells.

**Figure 4** demonstrates **a** binding affinity of gRBD, dRBD, and commercial RBDs of SARS-CoV-2 to ACE2, the receptor of SARS-CoV-2. The samples of 100 ng of plant-produced gRBD, dRBD, and commercially available **(A)** recombinant SARS-CoV-2 S protein RBD (amino acids Arg319-Phe541, with a C-terminal 8×His tag, expressed in 293E cells, cat. no. 793606, BioLegend, USA), or **(B)** SARS-CoV-2 spike protein (RBD) coronavirus active protein (amino acids Arg319-Phe541, produced in baculovirus-insect cells, cat. no. MBS2563882, MyBioSource, San Diego, CA, USA) were incubated on plates coated with ACE2. After incubation, anti-SARS-CoV-2 spike RBD polyclonal antibodies were added into each well and detected with rabbit IgG conjugated with HRP. Each point on the graph was derived from three replica for each dilution. Data are shown as mean ± standard error of the mean (SEM) of triplicates in each sample dilution. Statistical significance (*p* < 0.05) was calculated using the one-way ANOVA test with Tukey's multiple comparisons. p value for each group is shown in parentheses. ^{∗∗} *p* < 0.01, ^{∗∗∗} *p* < 0.001; OD-optical density; Kd-dissociation constant.

**Figure 5** demonstrates Immunogenicity of plant-produced RBD variants in mice. Mice (6-7-week-old Balb/c male) were immunized on days 0 and 21 IM with 5 µg (with alum) of plant-produced gRBD and dRBD using Alhydrogel as an adjuvant. IgG titers were determined by ELISA in sera collected on days 21 or 42 post-vaccination from mice immunized with plant-produced gRBD and dRBD. For immunogenicity analysis, 10² to 10⁸ dilutions of sera were assessed by ELISA. Endpoint titer refers to reciprocal serum dilutions that give a mean OD value four times greater than the pre-immune control samples. Control group received PBS with Alhydrogel. **(A)** Detection of the SARS-CoV-2 RBD-specific IgG in different dilutions of sera from day 21 or 42. **(B)** Detection of IgG titers in the murine serum with different dilutions specific to commercially available S protein, gRBD, dRBD at day 21 or 42. Each point on the graph was derived from three replicas for each dilution. Data are shown as mean ± standard error of the mean (SEM) of triplicates in each sample dilution. Statistical significance (*p* < 0.05) was calculated using the one-way ANOVA test with Tukey's multiple comparisons. ^{∗∗∗} *p* < 0.001 (n = 6 mice/group).

**Figure 6** demonstrates In vitro microneutralization assay of mouse sera against live SARS-CoV-2 in the Vero E6 cell line immunized with plant-produced gRBD or dRBD. In the assay, 32 to 1024 dilutions of the sera were used. Statistical significance (*p* < 0.05) was calculated using the one-way ANOVA test. ^{∗∗} *p <* 0.01; ^{∗∗∗} *p <* 0.001; n = 6 mice/group.

**Figure 7** demonstrates SDS-PAGE **(A),** Western blot **(B-D),** and glycan detection **(E)** analysis of purified plant-produced N protein. N protein from *N. benthamiana* plant was purified using anti-FLAG antibody resin. Membranes were probed with anti-Flag **(B,D)** or anti-N protein monoclonal antibody (human novel coronavirus nucleoprotein, 1-419 aa, monoclonal antibody, **(C)). (A)** Anti-FLAG antibody resin-purified N protein was analyzed by SDS-PAGE. N: 2 µg of plant-produced, purified N protein was loaded into a well. BSA standards: 1.0, 2.5, and 5.0 µg of BSA protein was loaded as a standard protein. M: color pre-stained protein standard (NEB, Ipswich, MA, USA). **(B)** Plant-produced N protein was run in reducing (R) or nonreducing condition (NR). Lanes: 25, 50, and 100 ng of plant-produced PNGase was loaded as a standard protein. **(C)** Anti-Flag column-purified N protein was analyzed by Western blot analysis using anti-N protein mAb. **(E)** Glycan detection analysis of anti-Flag purified N protein (N) in vivo co-expressed with Endo H (N + Endo H, in vivo). M-CandyCane glycoprotein molecular weight standards (Molecular Probes), 250 ng of each protein per lane. **(D))** Western blot analysis of the same sample using the anti-FLAG antibody.

**Figure 7** demonstrates SDS-PAGE **(A)** and Western blot **(B)** analysis of co-expression of RBD with N protein. **(A)** SDS-PAGE analysis of plant-produced, anti-Flag affinity column-purified N, RBD, and N+RBD proteins. N: plant-produced purified N protein; RBD: plant-produced purified RBD protein; RBD + N: RBD + N protein, purified from *N. benthamiana* plant, co-expressed with N and RBD constructs. BSA standards: 1.0 and 2.5 µg of BSA protein was loaded as a standard protein. M: color pre-stained protein standard (NEB, Ipswich, MA, USA). **(B)** Western blot analysis of plant-produced, anti-Flag affinity column-purified N, RBD, and N+RBD proteins. The proteins were loaded into wells as indicated; PNGase F: 25 or 50 ng of plant-produced PNGase F was used as a control protein.

**Figure 7** demonstrates Gel filtration chromatography and SDS-PAGE analysis of RBD, N, and N+RBD proteins, eluted from a Sephacryl^{®} S-200 HR column. **(A)** Profiles of plant-produced gRBD, N, and N+gRBD proteins, eluted from a Sephacryl^{®} S-200 HR column. The column was equilibrated with 50 mM phosphate buffer, pH 7.4, containing 150 mM NaCl. The plant-produced, FLAG affinity chromatography-purified gRBD, N, and N+gRBD proteins (0.25 mg) were loaded onto the column. Gel filtration was performed with ÄKTA start using a 60 cm × 16 mm column (cat. no. 19-5003-01, GE Healthcare, Chicago, IL, USA), packed with Sephacryl^{®} S-200 HR (cat. no. 17-0584-10, GE Healthcare). **(B)** SDS-PAGE analysis of plant-produced gRBD, N, and N+gRBD proteins eluted from Sephacryl^{®} S-200 HR column. gRBD: plant-produced RBD (~36 kDa); N: plant-produced N protein (~48 kDa); N+gRBD: antigen cocktail containing N protein (~48 kDa) and gRBD (~36 kDa); BSA stn: 1.0, 2.5, and 5.0 µg of BSA protein was loaded as a standard protein. M: color pre-stained protein standard (NEB, UK).

**Figure 8** demonstrates Stability of plant produced N protein. **(A)** Plant-produced, FLAG antibody affinity column-purified N protein was incubated at 37 °C for 24, 48, 72, and 96 h, and analyzed in SDS-PAGE. Lanes were loaded with ~2.0 µg of N protein. **(B)** Plant-produced, FLAG antibody affinity columnpurified N protein was stored at -80 °C for 6 months and then analyzed in SDS-PAGE M: color pre-stained protein standard.

**Figure 9** demonstrates Immunogenicity of plant-produced N protein and N+RBD in mice. First, 6-7 week old Balb/c male mice were immunized on IM with 5 µg of plant-produced N and N+RBD using Alhydrogel as an adjuvant on the 0th and 21st days. IgG titers of mice immunized with plant-produced N and N+RBD antigens were determined by ELISA from sera collected on the 21st and 42nd days after injection. For the detection of IgG titers, mouse sera on the 21st day and 42nd days were evaluated by 10² to 10⁸ serum dilutions. **(A)** Detection of specific IgG titers in 21st and 42nd day sera from mice immunized with N and N+RBD. **(B)** Detection of endpoint titers in sera with different dilutions specific to N and N+RBD on days 21 and 42. **(C)** Determination of specific IgG titer for N, RBD, and N+RBD antigens in mice immunized with plant-produced N+RBD. First, 6-7 week old Balb/c male mice were immunized on IM with 5 µg of plant-produced RBD and N+RBD using Alhydrogel as an adjuvant on the 0th and 21st days. Specific IgG titers of mice immunized with plant-produced N+RBD were determined by ELISA from sera collected on the 42nd day after injection. The ELISA plate was coated with 100 ng of plant-produced N, RBD, and N+RBD proteins. **(C)** (left) N-, RBD, and N+RBD-specific IgG titers on 42nd day sera from mice immunized with N+RBD. **(C)** (right) Detection of endpoint titers in mice sera immunized with N+RBD with different dilutions specific to N, RBD, and N+RBD on day 42. Endpoint titer was derived from reciprocal dilutions of sera, giving an OD value four times greater than mouse sera receiving PBS with Alhydrogel as a control sample. Data in the graph are shown as the mean ± standard error of the mean (SEM) of triplicates at each sample dilution. Statistical analysis was performed using Tukey's multiple comparison tests; ^{∗∗} *p <* 0.01, and ^{∗∗∗} *p <* 0.001 (*n* = 6 mice/group).

**Figure 10** demonstrates In vitro microneutralization assay of mouse sera against live SARS-CoV-2 in the Vero E6 cell line immunized with plant-produced RBD, N, or N+RBD proteins. In vitro microneutralization assay of 21st and 42nd day mouse sera immunized with plant-produced RBD and N+RBD (~2.7 µg + 2.3 µg, respectively) against live SARS-CoV-2 in the Vero E6 cell line as indicated. gRBD 2nd immunization (5 µg): in vitro microneutralization of sera from mice immunized with 5 µg of gRBD on day 42. The experiment was performed using 32 to 1024 dilutions of mouse sera collected on day 21 and day 42. One-way ANOVA Tukey's multiple comparison tests were used to calculate statistical significance (*n =* 6 mice/group); ^{∗∗∗} *p <* 0.001.

**Figure 11****.** In vitro qualitative determination of SARS-CoV-2 Spike Protein IgG in serum of patients who infected with COVID-19 and recovered. Kit with S1 antigen: Kit prepared based on plant produced S1 protein of SARS-CoV-2 (in this study). Elabbscience Euroimmun: Elabscience Euroimmun ELISA Kits (SARS-CoV-2 Spike Protein IgG ELISA Kit, Catalog number: E-EL-E602) used as a control kit for comarision with the current kit, developed in this study. Blood samples were diluted in 100 fold. HRP Conjugated anti-human IgG (MyBioSource Inc., cat. no. MBS440121) were used to develop the antigen-antibody-HRP conjugated secondary antibody complex. The optical density (OD) was measured spectrophotometrically at a wavelength of 450 nm on a plate reader.

### References

Bae, J.-L., Lee, J.-G., Kang, T.-J., Jang, H.-S., Jang, Y.-S. & Yang, M.-S. (2003) Vaccine 21 , 4052-4058. pmid: 12922142.
Cui, N. M., Einstein, J., Narasimhulu, S., Vergara, C. E. & Gelvin S. B. (1995) Plant J. 7 , 661-676.
Cong Y, Ulasli M, Schepers H, Mauthe M, V'kovski P, Kriegenburg F, Thiel V, de Haan C, Reggiori F (2020) Nucleocapsid protein recruitment to replication-transcription complexes plays a crucial role in coronaviral life cycle. J Virol 94(4):e01925-19. https://doi.org/10.1128/JVI.01925-19.
Kang, S.; Yang, M.; Hong, Z.; Zhang, L.; Huang, Z.; Chen, X.; He, S.; Zhou, Z.; Zhou, Z.; Chen, Q.; et al. Crystal structure of SARS-CoV-2 nucleocapsid protein RNA binding domain reveals potential unique drug targeting sites. Acta Pharm. Sin. B 2020, 10, 1228-1238. [CrossRef] [PubMed]
Diego-Martin, B.; González, B.; Vazquez-Vilar, M.; Selma, S.; Mateos-Fernández, R.; Gianoglio, S.; Fernandez-del-Carmen, A.;Orzáez, D. Pilot production of SARS-CoV-2 related proteins in plants: A proof of concept for rapid repurposing of indoor farms into biomanufacturing facilities. Front. Plant Sci. 2020, 11, 612781. [CrossRef]
Dutta NK, Mazumdar K, Gordy JT (2020) The nucleocapsid protein of SARS-CoV-2: a target for vaccine development. J Virol 94(13):e00647-20. https://doi.org/10.1128/JVI.00647-20.
Drosten et al. Identification of a novel coronavirus in patients with severe acute respiratory syndrome2003.N Engl J Med 2003 May 15;348(20):1967-76. doi: 10.1056/NEJMoa030747.
Holmes KV, Enjuanes L. 2003. Virology. The SARS coronavirus: Apostgenomic era. Science 300: 1377-1378.
Kang et al., 2020. Crystal structure of SARS-CoV-2 nucleocapsid protein RNA binding domain reveals potential unique drug targeting sites. Bio Rxiv. doi:
Grifoni, A.; Sidney, J.; Zhang, Y.; Scheuermann, R.H.; Peters, B.; Sette, A. A sequence homology and bioinformatic approach can predict candidate targets for immune responses to SARS-CoV-2. Cell Host Microbe 2020, 27, 671-680.
Lamphear, B. J., Jilka, J. M., Kesl, L., Welter, M., Howard, J. A. & Streatfield, S. J. (2004) Vaccine, 22 , 2420-2424. pmid: 15193404
Lan J. et al. (2020) Structure of the SARS-CoV-2 spike receptor-binding domain bound to the ACE2 receptor. Nature, 581, 215-22.
Leung, D.T.; Tam, F.C.; Ma, C.H.; Chan, P.K.; Cheung, J.L.; Niu, H.; Tam, J.S.; Lim, P.L. Antibody response of patients with severe
acute respiratory syndrome (SARS) targets the viral nucleocapsid. J. Infect. Dis. 2004, 190, 379-386.
Li HY, Ramalingam S, Chye ML. Accumulation of recombinant SARS-CoV spike protein in plant cytosol and chloroplasts indicate potential for development of plant-derived oral vaccines. Exp Biol Med (Maywood). 2006 Sep;231(8):1346-52. doi: 10.1177/153537020623100808.
Leung DT, Tam FC, Ma CH, Chan PK, Cheung JL, Niu H, Tam JS, LimPL. 2004. Antibody response of patients with severe acuterespiratory syndrome (SARS) targets the viral nucleocapsid. JInfect Dis 190:379-386.
Maharjan, P.M.; Choe, S. Plant-Based COVID-19 Vaccines: Current Status, Design, and Development Strategies of Candidate Vaccines. Vaccines 2021, 9, 992.
Mamedov T, Cicek K, Gulec B, Ungor R, Hasanova G. In vivo production of non-glycosylated recombinant proteins in Nicotiana benthamiana plants by co-expression with Endo-β-N-acetylglucosaminidase H (Endo H) of Streptomyces plicatus. PLoS One. 2017 Aug 21;12(8):e0183589. doi: 10.1371/journal.pone.0183589. eCollection 2017.
Mamedov T, Ghosh A, Jones RM, Mett V, Farrance CE, Musiychuk K, Horsey A, Yusibov V (2012) Production of non-glycosylated recombinant proteins in Nicotiana benthamiana plants by co-expressing bacterial PNGase F. Plant Biotechnol J 10(7):773-82.
Mamedov T, Chichester JA, Jones RM, Ghosh A, Coffin MV, Herschbach K, et al. (2016) Production of functionally active and immunogenic non-glycosylated protective antigen from Bacillus anthracis in Nicotiana benthamiana by co-expression with peptide-N-glycosidase F (PNGase F) of Flavobacterium meningosepticum. PLoS One 21; 11(4):e0153956.
Mamedov, T., Soylu, I.; Mammadova, G.; Hasanova, G. Sequence Analysis and Amino Acid Variations of Structural Proteins Deduced From Novel Coronavirus SARS-CoV-2 Strains, Isolated in Different Countries. Preprints 2020, 2020050026 (doi: 10.20944/preprints202005.0026.v1).
Mamedov T, Musayeva I, Acsora R, Gun N, Gulec B, Mammadova G, Cicek K, Hasanova G.Engineering, and production of functionally active human Furin in N. benthamiana plant: In vivo post-translational processing of target proteins by Furin in plants. PloS One. 2019a Mar 12;14(3):e0213438. doi: 10.1371/journal.pone.0213438. eCollection 2019.
Mamedov T, Cicek K, Miura K, Gulec B, Akinci A and Mammadova G, Hasanova G. A Plant-Produced in vivo deglycosylated full-length Pfs48/45 as a Transmission-Blocking Vaccine Candidate against malaria. Sci Rep 2019b. PMID 31285498.
Mamedov, T.; Soylu, I.; Mammadova, G.; Hasanova, G. Sequence Analysis and Amino Acid Variations of Structural Proteins Deduced From Novel Coronavirus SARS-CoV-2 Strains, Isolated in Different Countries. Preprints 2020, 2020050026.
Mamedov Tarlan, Damla Yuksel, Merve Ilgm, Irem Gurbuzaslan, Burcu Gulec, Hazel Yetiskin, Shaikh Terkis Islam Pavel Muhammet Ali Uygut 2, Aykut Ozdarendeli, Gulshan Mammadova, Deniz Say and Gulnara Hasanova. Plant-Produced Glycosylated and In Vivo Deglycosylated Receptor Binding Domain Proteins of SARS-CoV-2 Induce Potent Neutralizing Responses in Mice (2021) Viruses 13 (8), 1595
Mamedov T, Yuksel D, Ilgm M, Gürbüzaslan I, Gulec B, Mammadova G, ...Production and Characterization of Nucleocapsid and RBD Cocktail Antigens of SARS-CoV-2 in Nicotiana benthamiana Plant as a Vaccine Candidate against COVID-19 (2021). Vaccines 9 (11), 1337.
Sainsbury F, Thuenemann EC, Lomonossoff GP (2009) pEAQ: versatile expression vectors for easy and quick transient expression of heterologous proteins in plants. Plant Biotechnol 7(7):682±93. https://doi.org/10.1111/j.1467-7652.2009.00434.x PMID: 19627561
Pogrebnyak N, Golovkin M, Andrianov V, Spitsin S, Smirnov Y, Egolf R, Koprowski H. Severe acute respiratory syndrome (SARS) S protein production in plants development of recombinant vaccine. Proc Natl Acad Sci USA. 2005. PMID: 15956182
Production of in vivo n-deglycosylated recombinant proteins by co-expression with Endo H, Inventor Tarlan MAMMEDOV, WO2017081520A1, 2015-11-13.
Rattanapisit, K.; Shanmugaraj, B.; Manopwisedjaroen, S.; Purwono, P.B.; Siriwattananon, K.; Khorattanakulchai, N.; Hanittinan, O.; Boonyayothin,W.; Thitithanyanont, A.; Smith, D.R.; et al. Rapid production ofSARS-CoV-2 receptor binding domain (RBD) and spike specific monoclonal antibody CR3022 in Nicotiana Benthamiana. Sci. Rep. 2020, 10, 17698.
Shin, Y.J.; König-Beihammer, J.; Vavra, U.; Schwestka, J.; Kienzl, N.F.; Klausberger, M.; Laurent, E.; Grünwald-Gruber, C.; Vierlinger, K.; Hofner, M.; et al. N-Glycosylation of the SARS-CoV-2 Receptor Binding Domain Is Important for Functional Expression in Plants. Front. Plant Sci. 2021, 12, 689104.
Wrapp et. al. Cryo-EM structure of the 2019-nCoV spike in the prefusion conformation. Science 13 Mar 2020: Vol. 367, Issue 6483, pp. 1260-1263 DOI: 10.1126/science.abb2507.
Zhou, J. Y., Cheng, L. Q., Zheng, X. J., Gong, H., Shang, S. B. & Zhou, E. M. (2003) J. Virol. 77, 9090-9093. pmid:12885926
Zhou P, Yang XL, Wang XG, Hu B, Zhang L, Zhang W, et al. A pneumonia outbreak associated with a new coronavirus of probable bat origin. Nature. 2020a, 579 (7798): 270-273. doi:10.1038/s41586-020-2012-7. PMC 7095418. PMID 32015507.
Zhou Dapeng, Tian Xiaoxu, Qi Ruibing, Peng Chao, Zhang Wen. Identification of 22 N-glycosites on spike glycoprotein of SARS-CoV-2 and accessible surface glycopeptide motifs: implications for vaccination and antibody therapeutics. Glycobiology. 2020b. doi: 10.1093/glycob/cwaa052.PMID: 32518941

SEQUENCE LISTING

SEQ1:
<210> SEQ ID NO: 1
<211> Length: 1383
<212> Type: DNA (Spike gene of SARS–CoV-2)
<213> Organism: artificial
<223> Other information: recombinant DNA
<400> SEQUENCE 1

ATGGGATTTGTTTTGTTCTCTCAATTGCCATCTTTTCTTTTGGTTTCTACTCTTTTGCTT
TTCCTTGTTATTTCTCATTCTTGTAGAGCTATGTCTGATAATGGACCTCAGAATCAGA
GAAATGCTCCTAGAATTACTTTTGGAGGACCATCTGATTCTACTGGTTCTAATCAAA
ATGGTGAGAGGTCTGGAGCTAGGTCTAAGCAGAGAAGACCACAAGGTTTGCCAAAT
AATACTGCTTCTTGGTTTACAGCTCTTACTCAGCATGGAAAGGAGGATCTTAAGTTT
CCAAGGGGACAGGGTGTTCCAATTAATACTAATTCTTCTCCTGATGATCAGATTGGA
TATTATAGAAGGGCTACAAGAAGAATTAGGGGTGGAGATGGAAAGATGAAGGATTT
GTCTCCTAGATGGTATTTTTATTACTTGGGTACAGGACCTGAAGCTGGACTTCCATAC
GGAGCTAATAAGGATGGTATTATTTGGGTTGCTACAGAAGGAGCTCTTAATACACCT
AAGGATCATATTGGTACAAGAAATCCTGCTAATAATGCTGCTATTGTTTTGCAGCTT
CCACAGGGTACTACACTTCCAAAGGGATTTTATGCTGAGGGATCTAGAGGTGGTTCT
CAAGCTTCTTCTAGATCTTCTTCTAGGTCTAGAAATTCTTCTAGGAATTCTACTCCTG
GATCTTCTAGAGGAACATCTCCTGCTAGAATGGCTGGAAATGGTGGTGACGCTGCTC
TTGCTTTGCTTTTGCTTGATAGATTGAATCAGTTGGAGTCTAAGATGTCTGGTAAAGG
TCAGCAACAACAAGGTCAAACTGTTACAAAGAAGTCTGCTGCTGAGGCTTCTAAGA
AGCCTAGACAGAAGAGAACAGCTACTAAGGCTTATAATGTTACACAAGCTTTTGGT
AGAAGGGGTCCTGAACAGACACAAGGTAATTTTGGAGATCAAGAGTTGATTAGGCA
GGGTACAGATTACAAGCATTGGCCTCAGATTGCTCAGTTTGCTCCTTCTGCTTCTGCT
TTCTTTGGTATGTCTAGAATTGGTATGGAAGTTACTCCATCTGGTACATGGTTGACAT
ACACTGGTGCTATTAAGCTTGATGATAAGGATCCTAATTTCAAGGATCAAGTTATTC
TTCTTAACAAGCATATCGATGCTTATAAGACATTTCCACCAACAGAGCCTAAGAAGG

ATAAGAAGAAGAAGGCTGATGAGACACAAGCTTTGCCTCAAAGACAGAAGAAGCA
GCAGACTGTTACATTGTTGCCTGCTGCTGATCTTGATGATTTTCTAAGCAACTTCAG
CAGTCTATGTCTTCTGCTGATTCTACACAGGCTGATTACAAGGATGATGATGATAAG
GACGAACTTTAA

SEQ2:
210> SEQ ID NO: 2
<211> Length: 460
<212> Type: protein
<213> Organism: artificial sequence
<223> Other information: recombinant protein
<400> SEQUNCE 2
MetGlyPheValLeuPheSerGlnLeuProSerPheLeuLeuValSerThrLeuLeuLeu
PheLeuValIleSerHisSerCysArgAlaMetSerAspAsnGlyProGlnAsnGlnArg
AsnAlaProArgIleThrPheGlyGlyProSerAspSerThrGlySerAsnGlnAsnGly
GluArgSerGlyAlaArgSerLysGlnArgArgProGlnGlyLeuProAsnAsnThrAla
SerTrpPheThrAlaLeuThrGlnHisGlyLysGluAspLeuLysPheProArgGlyGln
GlyValProIleAsnThrAsnSerSerProAspAspGlnIleGlyTyrTyrArgArgAla
ThrArgArgIleArgGlyGlyAspGlyLysMetLysAspLeuSerProArgTrpTyrPhe
TyrTyrLeuGlyThrGlyProGluAlaGlyLeuProTyrGlyAlaAsnLysAspGlyIle
IleTrpValAlaThrGluGlyAlaLeuAsnThrProLysAspHisIleGlyThrArgAsn
ProAlaAsnAsnAlaAlaIleValLeuGlnLeuProGlnGlyThrThrLeuProLysGly
PheTyrAlaGluGlySerArgGlyGlySerGlnAlaSerSerArgSerSerSerArgSer
ArgAsnSerSerArgAsnSerThrProGlySerSerArgGlyThrSerProAlaArgMet
AlaGlyAsnGlyGlyAspAlaAlaLeuAlaLeuLeuLeuLeuAspArgLeuAsnGlnLeu
GluSerLysMetSerGlyLysGlyGlnGlnGlnGlnGlyGlnThrValThrLysLysSer
AlaAlaGluAlaSerLysLysProArgGlnLysArgThrAlaThrLysAlaTyrAsnVal
ThrGlnAlaPheGlyArgArgGlyProGluGlnThrGlnGlyAsnPheGlyAspGlnGlu
LeuIleArgGlnGlyThrAspTyrLysHisTrpProGlnIleAlaGlnPheAlaProSer
AlaSerAlaPhePheGlyMetSerArgIleGlyMetGluValThrProSerGlyThrTrp
LeuThrTyrThrGlyAlaIleLysLeuAspAspLysAspProAsnPheLysAspGlnVal

IleLeuLeuAsnLysHisIleAspAlaTyrLysThrPheProProThrGluProLysLys
AspLysLysLysLysAlaAspGluThrGlnAlaLeuProGlnArgGlnLysLysGlnGln
ThrValThrLeuLeuProAlaAlaAspLeuAspAspPheSerLysGlnLeuGlnGlnSer
MetSerSerAlaAspSerThrGlnAlaAspTyrLysAspAspAspAspLysAspGluLeu

SEQ3
<210> SEQ ID NO: 3
<211> Length: 945
<212> Type: DNA (Spike gene of SARS–CoV-2)
<213> Organism: artificial
<223> Other information: recombinant DNA
<400> SEQUENCE 3
RBD sequence (319-591 AA surface glycoprotein, ACCESSION MN985325):

ATGGGATTCGTTCTCTTCTCTCAGCTTCCATCATTCCTTCTTGTTAGTACACTTCTTCT
CTTCTTGGTTATTAGTCATTCTTGTAGGGCTAGGGTTCAGCCTACAGAATCTATTGTT
AGGTTCCCTAATATTACTAACTTGTGTCCATTCGGTGAAGTGTTCAATGCTACTAGAT
TCGCTTCTGTGTATGCTTGGAATAGAAAGAGGATTAGTAATTGTGTTGCTGATTATTC
TGTGTTGTATAATTCTGCTTCATTCTCTACATTCAAGTGTTACGGAGTTAGTCCTACT
AAGTTGAATGATCTCTGCTTCACTAATGTGTATGCTGATTCATTCGTTATTAGAGGTG
ATGAAGTTAGGCAGATTGCTCCAGGTCAAACAGGTAAGATTGCTGATTATAATTACA
AGTTGCCTGATGACTTCACAGGTTGTGTTATTGCTTGGAATTCTAATAATCTCGATTC
TAAGGTTGGTGGAAATTACAATTATCTCTATAGATTGTTCAGGAAGTCTAATCTTAA
GCCATTCGAAAGAGATATTAGTACAGAAATCTATCAGGCTGGATCTACTCCATGTAA
TGGTGTTGAGGGATTCAATTGTTACTTCCCACTTCAGTCTTACGGATTCCAACCTACA
AATGGAGTTGGATATCAACCTTATAGAGTTGTTGTTCTTAGCTTCGAACTTCTTCATG
CTCCAGCTACTGTGTGTGGACCTAAGAAGTCTACAAATCTTGTTAAGAATAAGTGCG
TTAACTTCAACTTCAATGGTCTTACTGGTACTGGTGTTCTTACTGAATCTAATAAGAA
GTTCTTGCCATTCCAGCAATTCGGTAGAGATATTGCTGATACAACTGATGCTGTTAG

AGATCCACAAACATTGGAGATTCTTGATATTACACCATGTTCTGATTACAAGGATGA
TGATGATAAGGATGAGCTTTGA

SEQ4:
<210> SEQ ID NO: 4
<211> Length: 314
<212> Type: protein (Spike Protein of SARS–CoV-2)
<213> Organism: artificial sequence
<223> Other information: recombinant protein
<400> SEQUNCE 4

MetGlyPheValLeuPheSerGlnLeuProSerPheLeuLeuValSerThrLeuLeuLeu
PheLeuValIleSerHisSerCysArgAlaArgValGlnProThrGluSerIleValArg
PheProAsnIleThrAsnLeuCysProPheGlyGluValPheAsnAlaThrArgPheAla
SerValTyrAlaTrpAsnArgLysArgIleSerAsnCysValAlaAspTyrSerValLeu
TyrAsnSerAlaSerPheSerThrPheLysCysTyrGlyValSerProThrLysLeuAsn
AspLeuCysPheThrAsnValTyrAlaAspSerPheValIleArgGlyAspGluValArg
GlnIleAlaProGlyGlnThrGlyLysIleAlaAspTyrAsnTyrLysLeuProAspAsp
PheThrGlyCysValIleAlaTrpAsnSerAsnAsnLeuAspSerLysValGlyGlyAsn
TyrAsnTyrLeuTyrArgLeuPheArgLysSerAsnLeuLysProPheGluArgAspIle
SerThrGluIleTyrGlnAlaGlySerThrProCysAsnGlyValGluGlyPheAsnCys
TyrPheProLeuGlnSerTyrGlyPheGlnProThrAsnGlyValGlyTyrGlnProTyr
ArgValValValLeuSerPheGluLeuLeuHisAlaProAlaThrValCysGlyProLys
LysSerThrAsnLeuValLysAsnLysCysValAsnPheAsnPheAsnGlyLeuThrGly
ThrGlyValLeuThrGluSerAsnLysLysPheLeuProPheGlnGlnPheGlyArgAsp
IleAlaAspThrThrAspAlaValArgAspProGlnThrLeuGluIleLeuAspIleThr
ProCysSerAspTyrLysAspAspAspAspLysAspGluLeu
<210> SEQ ID NO: 5
<211> Length: 942
<212> Type: DNA
<213> Organism: artificial

<223> Other information: recombinant DNA
<400> SEQUENCE 5

ATGGGTTTCGTGCTGTTCAGCCAGCTGCCTTCTTTCCTTCTTGTGTCTACCCTTCTGCT
GTTCCTGGTGATCTCTCATTCTTGTAGGGCTCCAGCTCCTGCTCCTGTTAAGCAAGGT
CCTACTTCTGTGGCTTACGTTGAGGTGAACAACAACAGCATGCTGAACGTGGGAAA
GTACACCCTTGCTGATGGTGGTGGTAACGCTTTCGATGTGGCTGTGATTTTCGCTGCT
AACATCAACTACGATACCGGTACTAAGACCGCTTACCTGCACTTCAATGAGAACGTG
CAGAGGGTGTTGGATAACGCTGTGACTCAGATTAGGCCTCTTCAGCAGCAGGGTATT
AAGGTGCTGCTTTCTGTGCTTGGTAACCACCAGGGTGCTGGTTTCGCTAATTTTCCTA
GTCAGCAGGCTGCTTCCGCTTTCGCTAAGCAACTTTCTGATGCTGTGGCTAAGTACG
GTCTGGATGGTGTGGATTTCGATGATGAGTACGCTGAGTACGGTAACAACGGTACTG
CTCAGCCTAACGATAGCTCTTTCGTGCATCTTGTGACCGCTCTGAGGGCTAACATGC
CTGATAAGATCATCAGCCTTTACAACATCGGTCCTGCTGCTTCCAGGCTTTCTTACGG
TGGTGTTGATGTGAGCGATAAGTTCGATTACGCTTGGAACCCTTACTACGGAACCTG
GCAAGTTCCTGGTATTGCTTTGCCTAAGGCTCAGCTTTCTCCAGCTGCTGTTGAGATT
GGTAGGACCTCTAGGTCTACCGTGGCTGATCTTGCTAGAAGGACTGTGGATGAGGGT
TACGGTGTGTACCTTACCTACAACCTGGATGGTGGTGATAGGACCGCTGATGTGTCT
GCTTTCACCAGAGAGCTTTACGGTTCTGAGGCTGTGAGGACCCCTGATTACAAGGAC
GATGATGATAAGGATGAGCTGTAG

<210> SEQ ID NO: 6
<211> Length: 313
<212> Type: protein
<213> Organism: artificial sequence
<223> Other information: recombinant protein
<400> SEQUNCE 6

MetGlyPheValLeuPheSerGlnLeuProSerPheLeuLeuValSerThrLeuLeuLeu
PheLeuValIleSerHisSerCysArgAlaProAlaProAlaProValLysGlnGlyPro
ThrSerValAlaTyrValGluValAsnAsnAsnSerMetLeuAsnValGlyLysTyrThr

LeuAlaAspGlyGlyGlyAsnAlaPheAspValAlaValIlePheAlaAlaAsnIleAsn
TyrAspThrGlyThrLysThrAlaTyrLeuHisPheAsnGluAsnValGlnArgValLeu
AspAsnAlaValThrGlnIleArgProLeuGlnGlnGlnGlyIleLysValLeuLeuSer
ValLeuGlyAsnHisGlnGlyAlaGlyPheAlaAsnPheProSerGlnGlnAlaAlaSer
AlaPheAlaLysGlnLeuSerAspAlaValAlaLysTyrGlyLeuAspGlyValAspPhe
AspAspGluTyrAlaGluTyrGlyAsnAsnGlyThrAlaGlnProAsnAspSerSerPhe
ValHisLeuValThrAlaLeuArgAlaAsnMetProAspLysIleIleSerLeuTyrAsn
IleGlyProAlaAlaSerArgLeuSerTyrGlyGlyValAspValSerAspLysPheAsp
TyrAlaTrpAsnProTyrTyrGlyThrTrpGlnValProGlyIleAlaLeuProLysAla
GlnLeuSerProAlaAlaValGluIleGlyArgThrSerArgSerThrValAlaAspLeu
AlaArgArgThrValAspGluGlyTyrGlyValTyrLeuThrTyrAsnLeuAspGlyGly
AspArgThrAlaAspValSerAlaPheThrArgGluLeuTyrGlySerGluAlaValArg
ThrProAspTyrLysAspAspAspAspLysAspGluLeu

< 210 > SEQ ID NO 7
< 211 > LENGTH : 30
< 212 > TYPE : PRT
< 213 > ORGANISM : Artificial Sequence
< 220 > FEATURE : < 223 > OTHER INFORMATION : Nicotiana benthamiana recombinant
protein
< 400 > SEQUENCE : 7
Met Gly Phe Val Leu Phe Ser Gin Leu Pro Ser Phe Leu Leu Val Ser 1 5 10 15 Thr Leu Leu Leu
Phe Leu Val Ile Ser His Ser Cys Arg Ala

210 > SEQ ID NO 8
< 211 > LENGTH : 8
< 212 > TYPE : PRT
< 213 > ORGANISM : Artificial Sequence
< 220 > FEATURE :
< 223 > OTHER INFORMATION : synthetic sequence

< 400 > SEQUENCE : 8
AspTyrLysAspAspAspAspLys

< 210 > SEQ ID NO 9
< 211 > LENGTH : 4
< 212 > TYPE : PRT
< 213 > ORGANISM : Artificial Sequence
< 220 > FEATURE :
< 223 > OTHER INFORMATION : synthetic sequence
< 400 > SEQUENCE : 9
Lys Asp Glu Leu

\

SEQ1:
<210> SEQ ID NO: 1
<211> Length: 1383
<212> Type: DNA (Spike gene of SARS\226CoV-2)
<213> Organism: artificial
<223> Other information: recombinant DNA
<400> SEQUENCE 1

ATGGGATTTGTTTTGTTCTCTCAATTGCCATCTTTTCTTTTGGTTTCTACTCTTTTGCTTTTCCTTGTTATTTCTCA
TTCTTGTAGAGCTATGTCTGATAATGGACCTCAGAATCAGAGAAATGCTCCTAGAATTACTTTTGGAGGACCATCTG
ATTCTACTGGTTCTAATCAAAATGGTGAGAGGTCTGGAGCTAGGTCTAAGCAGAGAAGACCACAAGGTTTGCCAAAT
AATACTGCTTCTTGGTTTACAGCTCTTACTCAGCATGGAAAGGAGGATCTTAAGTTTCCAAGGGGACAGGGTGTTCC
AATTAATACTAATTCTTCTCCTGATGATCAGATTGGATATTATAGAAGGGCTACAAGAAGAATTAGGGGTGGAGATG
GAAAGATGAAGGATTTGTCTCCTAGATGGTATTTTTATTACTTGGGTACAGGACCTGAAGCTGGACTTCCATACGGA
GCTAATAAGGATGGTATTATTTGGGTTGCTACAGAAGGAGCTCTTAATACACCTAAGGATCATATTGGTACAAGAAA
TCCTGCTAATAATGCTGCTATTGTTTTGCAGCTTCCACAGGGTACTACACTTCCAAAGGGATTTTATGCTGAGGGAT
CTAGAGGTGGTTCTCAAGCTTCTTCTAGATCTTCTTCTAGGTCTAGAAATTCTTCTAGGAATTCTACTCCTGGATCT
TCTAGAGGAACATCTCCTGCTAGAATGGCTGGAAATGGTGGTGACGCTGCTCTTGCTTTGCTTTTGCTTGATAGATT
GAATCAGTTGGAGTCTAAGATGTCTGGTAAAGGTCAGCAACAACAAGGTCAAACTGTTACAAAGAAGTCTGCTGCTG
AGGCTTCTAAGAAGCCTAGACAGAAGAGAACAGCTACTAAGGCTTATAATGTTACACAAGCTTTTGGTAGAAGGGGT
CCTGAACAGACACAAGGTAATTTTGGAGATCAAGAGTTGATTAGGCAGGGTACAGATTACAAGCATTGGCCTCAGAT
TGCTCAGTTTGCTCCTTCTGCTTCTGCTTTCTTTGGTATGTCTAGAATTGGTATGGAAGTTACTCCATCTGGTACAT
GGTTGACATACACTGGTGCTATTAAGCTTGATGATAAGGATCCTAATTTCAAGGATCAAGTTATTCTTCTTAACAAG
CATATCGATGCTTATAAGACATTTCCACCAACAGAGCCTAAGAAGGATAAGAAGAAGAAGGCTGATGAGACACAAGC
TTTGCCTCAAAGACAGAAGAAGCAGCAGACTGTTACATTGTTGCCTGCTGCTGATCTTGATGATTTTTCTAAGCAAC
TTCAGCAGTCTATGTCTTCTGCTGATTCTACACAGGCTGATTACAAGGATGATGATGATAAGGACGAACTTTAA

SEQ2:
210> SEQ ID NO: 2
<211> Length: 460
<212> Type: protein
<213> Organism: artificial sequence
<223> Other information: recombinant protein
<400> SEQUNCE 2
MetGlyPheValLeuPheSerGlnLeuProSerPheLeuLeuValSerThrLeuLeuLeu
PheLeuValIleSerHisSerCysArgAlaMetSerAspAsnGlyProGlnAsnGlnArg
AsnAlaProArgIleThrPheGlyGlyProSerAspSerThrGlySerAsnGlnAsnGly
GluArgSerGlyAlaArgSerLysGlnArgArgProGlnGlyLeuProAsnAsnThrAla
SerTrpPheThrAlaLeuThrGlnHisGlyLysGluAspLeuLysPheProArgGlyGln
GlyValProIleAsnThrAsnSerSerProAspAspGlnIleGlyTyrTyrArgArgAla
ThrArgArgIleArgGlyGlyAspGlyLysMetLysAspLeuSerProArgTrpTyrPhe
TyrTyrLeuGlyThrGlyProGluAlaGlyLeuProTyrGlyAlaAsnLysAspGlyIle
IleTrpValAlaThrGluGlyAlaLeuAsnThrProLysAspHisIleGlyThrArgAsn
ProAlaAsnAsnAlaAlaIleValLeuGlnLeuProGlnGlyThrThrLeuProLysGly
PheTyrAlaGluGlySerArgGlyGlySerGlnAlaSerSerArgSerSerSerArgSer
ArgAsnSerSerArgAsnSerThrProGlySerSerArgGlyThrSerProAlaArgMet
AlaGlyAsnGlyGlyAspAlaAlaLeuAlaLeuLeuLeuLeuAspArgLeuAsnGlnLeu
GluSerLysMetSerGlyLysGlyGlnGlnGlnGlnGlyGlnThrValThrLysLysSer
AlaAlaGluAlaSerLysLysProArgGlnLysArgThrAlaThrLysAlaTyrAsnVal
ThrGlnAlaPheGlyArgArgGlyProGluGlnThrGlnGlyAsnPheGlyAspGlnGlu
LeuIleArgGlnGlyThrAspTyrLysHisTrpProGlnIleAlaGlnPheAlaProSer
AlaSerAlaPhePheGlyMetSerArgIleGlyMetGluValThrProSerGlyThrTrp
LeuThrTyrThrGlyAlaIleLysLeuAspAspLysAspProAsnPheLysAspGlnVal
IleLeuLeuAsnLysHisIleAspAlaTyrLysThrPheProProThrGluProLysLys
AspLysLysLysLysAlaAspGluThrGlnAlaLeuProGlnArgGlnLysLysGlnGln
ThrValThrLeuLeuProAlaAlaAspLeuAspAspPheSerLysGlnLeuGlnGlnSer
MetSerSerAlaAspSerThrGlnAlaAspTyrLysAspAspAspAspLysAspGluLeu

SEQ3
<210> SEQ ID NO: 3
<211> Length: 945

<212>  Type: DNA (Spike gene of SARS\226CoV-2)
<213>  Organism: artificial
<223>  Other information: recombinant DNA
<400> SEQUENCE 3
RBD sequence (319-591 AA surface glycoprotein,  ACCESSION   MN985325):

ATGGGATTCGTTCTCTTCTCTCAGCTTCCATCATTCCTTCTTGTTAGTACACTTCTTCTCTTCTTGGTTATTAGTCA
TTCTTGTAGGGCTAGGGTTCAGCCTACAGAATCTATTGTTAGGTTCCCTAATATTACTAACTTGTGTCCATTCGGTG
AAGTGTTCAATGCTACTAGATTCGCTTCTGTGTATGCTTGGAATAGAAAGAGGATTAGTAATTGTGTTGCTGATTAT
TCTGTGTTGTATAATTCTGCTTCATTCTCTACATTCAAGTGTTACGGAGTTAGTCCTACTAAGTTGAATGATCTCTG
CTTCACTAATGTGTATGCTGATTCATTCGTTATTAGAGGTGATGAAGTTAGGCAGATTGCTCCAGGTCAAACAGGTA
AGATTGCTGATTATAATTACAAGTTGCCTGATGACTTCACAGGTTGTGTTATTGCTTGGAATTCTAATAATCTCGAT
TCTAAGGTTGGTGGAAATTACAATTATCTCTATAGATTGTTCAGGAAGTCTAATCTTAAGCCATTCGAAAGAGATAT
TAGTACAGAAATCTATCAGGCTGGATCTACTCCATGTAATGGTGTTGAGGGATTCAATTGTTACTTCCCACTTCAGT
CTTACGGATTCCAACCTACAAATGGAGTTGGATATCAACCTTATAGAGTTGTTGTTCTTAGCTTCGAACTTCTTCAT
GCTCCAGCTACTGTGTGTGGACCTAAGAAGTCTACAAATCTTGTTAAGAATAAGTGCGTTAACTTCAACTTCAATGG
TCTTACTGGTACTGGTGTTCTTACTGAATCTAATAAGAAGTTCTTGCCATTCCAGCAATTCGGTAGAGATATTGCTG
ATACAACTGATGCTGTTAGAGATCCACAAACATTGGAGATTCTTGATATTACACCATGTTCTGATTACAAGGATGAT
GATGATAAGGATGAGCTTTGA

SEQ4:
<210>  SEQ ID NO: 4
<211>   Length: 314
<212>  Type: protein (Spike Protein of SARS\226CoV-2)
<213>  Organism: artificial sequence
<223>  Other information: recombinant protein
<400>  SEQUNCE 4

MetGlyPheValLeuPheSerGlnLeuProSerPheLeuLeuValSerThrLeuLeuLeu
PheLeuValIleSerHisSerCysArgAlaArgValGlnProThrGluSerIleValArg
PheProAsnIleThrAsnLeuCysProPheGlyGluValPheAsnAlaThrArgPheAla
SerValTyrAlaTrpAsnArgLysArgIleSerAsnCysValAlaAspTyrSerValLeu
TyrAsnSerAlaSerPheSerThrPheLysCysTyrGlyValSerProThrLysLeuAsn
AspLeuCysPheThrAsnValTyrAlaAspSerPheValIleArgGlyAspGluValArg
GlnIleAlaProGlyGlnThrGlyLysIleAlaAspTyrAsnTyrLysLeuProAspAsp
PheThrGlyCysValIleAlaTrpAsnSerAsnAsnLeuAspSerLysValGlyGlyAsn
TyrAsnTyrLeuTyrArgLeuPheArgLysSerAsnLeuLysProPheGluArgAspIle
SerThrGluIleTyrGlnAlaGlySerThrProCysAsnGlyValGluGlyPheAsnCys
TyrPheProLeuGlnSerTyrGlyPheGlnProThrAsnGlyValGlyTyrGlnProTyr
ArgValValValLeuSerPheGluLeuLeuHisAlaProAlaThrValCysGlyProLys
LysSerThrAsnLeuValLysAsnLysCysValAsnPheAsnPheAsnGlyLeuThrGly
ThrGlyValLeuThrGluSerAsnLysLysPheLeuProPheGlnGlnPheGlyArgAsp
IleAlaAspThrThrAspAlaValArgAspProGlnThrLeuGluIleLeuAspIleThr
ProCysSerAspTyrLysAspAspAspAspLysAspGluLeu
<210>  SEQ ID NO: 5
<211>  Length: 942
<212>  Type: DNA
<213>  Organism: artificial
<223>  Other information: recombinant DNA
<400>  SEQUENCE 5

ATGGGTTTCGTGCTGTTCAGCCAGCTGCCTTCTTTCCTTCTTGTGTCTACCCTTCTGCTGTTCCTGGTGATCTCTCA
TTCTTGTAGGGCTCCAGCTCCTGCTCCTGTTAAGCAAGGTCCTACTTCTGTGGCTTACGTTGAGGTGAACAACAACA
GCATGCTGAACGTGGGAAAGTACACCCTTGCTGATGGTGGTGGTAACGCTTTCGATGTGGCTGTGATTTTCGCTGCT
AACATCAACTACGATACCGGTACTAAGACCGCTTACCTGCACTTCAATGAGAACGTGCAGAGGGTGTTGGATAACGC
TGTGACTCAGATTAGGCCTCTTCAGCAGCAGGGTATTAAGGTGCTGCTTTCTGTGCTTGGTAACCACCAGGGTGCTG
GTTTCGCTAATTTTCCTAGTCAGCAGGCTGCTTCCGCTTTCGCTAAGCAACTTTCTGATGCTGTGGCTAAGTACGGT
CTGGATGGTGTGGATTTCGATGATGAGTACGCTGAGTACGGTAACAACGGTACTGCTCAGCCTAACGATAGCTCTTT
CGTGCATCTTGTGACCGCTCTGAGGGCTAACATGCCTGATAAGATCATCAGCCTTTACAACATCGGTCCTGCTGCTT
CCAGGCTTTCTTACGGTGGTGTTGATGTGAGCGATAAGTTCGATTACGCTTGGAACCCTTACTACGGAACCTGGCAA
GTTCCTGGTATTGCTTTGCCTAAGGCTCAGCTTTCTCCAGCTGCTGTTGAGATTGGTAGGACCTCTAGGTCTACCGT
GGCTGATCTTGCTAGAAGGACTGTGGATGAGGGTTACGGTGTGTACCTTACCTACAACCTGGATGGTGGTGATAGGA
CCGCTGATGTGTCTGCTTTCACCAGAGAGCTTTACGGTTCTGAGGCTGTGAGGACCCCTGATTACAAGGACGATGAT

GATAAGGATGAGCTGTAG

<210>  SEQ ID NO: 6
<211>   Length: 313
<212>  Type: protein
<213>  Organism: artificial sequence
<223>  Other information: recombinant protein
<400>  SEQUNCE 6

MetGlyPheValLeuPheSerGlnLeuProSerPheLeuLeuValSerThrLeuLeuLeu
PheLeuValIleSerHisSerCysArgAlaProAlaProAlaProValLysGlnGlyProThrSerValAlaTyrVa
lGluValAsnAsnAsnSerMetLeuAsnValGlyLysTyrThr
LeuAlaAspGlyGlyGlyAsnAlaPheAspValAlaValIlePheAlaAlaAsnIleAsn
TyrAspThrGlyThrLysThrAlaTyrLeuHisPheAsnGluAsnValGlnArgValLeu
AspAsnAlaValThrGlnIleArgProLeuGlnGlnGlyIleLysValLeuLeuSer
ValLeuGlyAsnHisGlnGlyAlaGlyPheAlaAsnPheProSerGlnGlnAlaAlaSer
AlaPheAlaLysGlnLeuSerAspAlaValAlaLysTyrGlyLeuAspGlyValAspPhe
AspAspGluTyrAlaGluTyrGlyAsnAsnGlyThrAlaGlnProAsnAspSerSerPhe
ValHisLeuValThrAlaLeuArgAlaAsnMetProAspLysIleIleSerLeuTyrAsn
IleGlyProAlaAlaSerArgLeuSerTyrGlyGlyValAspValSerAspLysPheAsp
TyrAlaTrpAsnProTyrTyrGlyThrTrpGlnValProGlyIleAlaLeuProLysAla
GlnLeuSerProAlaAlaValGluIleGlyArgThrSerArgSerThrValAlaAspLeu
AlaArgArgThrValAspGluGlyTyrGlyValTyrLeuThrTyrAsnLeuAspGlyGly
AspArgThrAlaAspValSerAlaPheThrArgGluLeuTyrGlySerGluAlaValArg
ThrProAspTyrLysAspAspAspAspLysAspGluLeu


< 210 > SEQ ID NO 7
 < 211 > LENGTH : 30
< 212 > TYPE : PRT
< 213 > ORGANISM : Artificial Sequence
< 220 > FEATURE : < 223 > OTHER INFORMATION : Nicotiana benthamiana
recombinant protein
 < 400 > SEQUENCE : 7
Met Gly Phe Val Leu Phe Ser Gin Leu Pro Ser Phe Leu Leu Val Ser 1 5 10 15
Thr Leu Leu Leu Phe Leu Val Ile Ser His Ser Cys Arg Ala

210 > SEQ ID NO 8
< 211 > LENGTH : 8
< 212 > TYPE : PRT
< 213 > ORGANISM : Artificial Sequence
< 220 > FEATURE :
 < 223 > OTHER INFORMATION : synthetic sequence
< 400 > SEQUENCE : 8
AspTyrLysAspAspAspAspLys


< 210 > SEQ ID NO 9
< 211 > LENGTH : 4
< 212 > TYPE : PRT
< 213 > ORGANISM : Artificial Sequence
< 220 > FEATURE :
 < 223 > OTHER INFORMATION : synthetic sequence
< 400 > SEQUENCE : 9
Lys Asp Glu Leu

\

## Claims

1. A first nucleic acid comprising a first nucleotide sequence encoding the immunoactive polypeptide a SARC-CoV-2 nucleocapsid (N) protein, having the nucleic acid sequence of SEQ ID NO 1 or 2, wherein the first nucleotide sequence is operable linked to a promoter such that when the promoter is activated, the Nucleocapsid (N) polypeptide is expressed;
wherein the first nucleic acids is introduced into the cell via an Agrobacterium construct

2. A second nucleic acid comprising a nucleotide sequence encoding the immunoactive polypeptide, Receptor Binding Domain (RBD) of Spike protein of SARS-CoV-2 having the nucleic acid sequence of SEQ ID NO: 3 or 4, wherein the second nucleotide sequence is operable linked to a promoter such that when the promoter is activated, the polypeptide of interest, RBD protein, is expressed;
wherein the second nucleic acids is introduced into the cell via an Agrobacterium construct

3. A method for generating a multi-antigen, cocktail antigens of SARC-CoV-2 as a vaccine candidate by comprising the steps of:
(a) a first nucleic acid comprising a first nucleotide sequence encoding a SARC-CoV-2 nucleocapsid (N) protein, having the nucleic acid sequence of SEQ ID NO: 1 or 2, wherein the first nucleotide sequence is operable linked to a promoter such that when the promoter is activated, the nucleocapsid (N) protein polypeptide is expressed; and
(b) a second nucleic acid comprising a nucleotide sequence encoding the Receptor Binding Domain (RBD) of Spike protein of SARS-CoV-2 having the nucleic acid sequence of SEQ ID NO: 3 or 4, wherein the second nucleotide sequence is operable linked to a promoter such that when the promoter is activated, the polypeptide of interest, RBD polypeptide, is expressed;
(c) Co-expressing the first nucleic acid and the second nucleic acid in the eukaryotic cell, especially in plant cell or yeasts cell, a multi-antigen, a cocktail antigen of SARS-CoV-2 polypeptides comprising N protein and RBD (N+RBD) polypeptides are generated.
wherein the first and second nucleic acids are introduced into the cell via an Agrobacterium construct

4. A method of producing a N-deglycosylated of RBD, wherein the method comprising:
(a) a first nucleic acid comprising a second nucleotide sequence encoding immunoactive highly soluble and functional active polypeptide, a SARC-CoV-2 RBD protein, having the nucleic acid sequence of SEQ ID NO: 3 or 4, wherein the first nucleotide sequence is operable linked to a promoter such that when the promoter is activated, the RBD polypeptide is expressed;
(b) second nucleic acid comprising a first nucleotide sequence encoding bacterial Endo H (Endo-β-N-acetylglucosaminidase H, Endo H, EC3.2.1.96), having the nucleic acid sequence of SEQ ID NO: 5 or 6, wherein the first nucleotide sequence is operable linked to a promoter such that when the promoter is activated, the Endo H protein polypeptide is expressed; and
(c) Co-expressing the first nucleic acid and the second nucleic acid in the plant cell to generate the deglycosylated RBD polypeptide
wherein the first and second nucleic acids are introduced into the cell via an Agrobacterium construct

5. The method of claim 3, the first nucleotide sequence is Spike protein of SARC-CoV-2, including S1 domain

6. The method of claim 3, the first and second nucleotide sequences are polypeptides immunoactive in humans

7. The method of claim 4, wherein the polypeptide exhibited a stronger binding to Angiotensin-Converting Enzyme 2 (ACE2), a receptor of SARC-CoV or SARS-CoV-2, and also mice immunized with this antigen has increased neutralizing activity against SARS-CoV-2 compared with its glycosylated counterpart

8. The method of claim 4, the cocktail antigen elicited high-titer functional antibodies compared to RBD or N proteins

9. The method of claim 3, where the approach can be applied to existing COVID-19 vaccines, in particular mRNA, DNA, viral vectors, and other types of vaccines, to be effective toward new emerging variants

10. A product produced by expression of N protein for using as a vaccine against COVID-19

11. A product produced by expression of RBD protein for using as a vaccine against COVID-19

12. A product, a cocktail antigens, produced by co-expression of RBD protein and N protein for using as a vaccine against COVID-19

13. A product, a cocktail antigens, produced by co-expression of Spike protein including S1 domain protein and N protein for using as a vaccine against COVID-19

14. A product, deglycosylated RBD of SARS-CoV-2 produced by co-expression of RBD protein and Endo H for using as a vaccine against COVID-19

15. A product, a cocktail antigens, produced by co-expression of two or more polypeptides immunoactive in humans
